# EUROPEAN PATENT SPECIFICATION

(11) **EP 1 469 882 B1**
(45) Date of publication and mention of the grant of the patent: **25.08.2010**
(21) Application number: 03700950.3
(22) Date of filing: 28.01.2003
(51) Int. Cl.: A61K 39/395

(54) **TREATMENT OF NERVOUS SYSTEM DISEASES WITH GOAT SERUM**
BEHANDLUNG VON KRANKHEITEN DES NERVENSYSTEMS MIT ZIEGENSERUM
TRAITEMENT DE MALADIES DU SYSTEME NERVEUX AVEC UN SERUM DE CHEVRE

(30) Priority: 28.01.2002 GB 0201896; 02.07.2002 WO PCT/GB02/03037
(43) Date of publication of application: 27.10.2004
(73) Proprietor: Aimsco Limited, East Sussex BN21 4RL (GB)
(72) Inventor: HEENEY, Jonathan, 2275 BX Voorburg (NL); DALGLEISH, Angus G., Cheam, Surrey SU12 7PP (GB); WHITE, Stanley D. T., Hardwick, MA 01037 (US)
(74) Representative: Williams, Gareth Owen
(86) International application number: PCT/GB2003/000342
(87) International publication number: WO 2003/064472

(56) References cited:
- WO-A-00/12560
- WO-A-00/71160
- WO-A-02/07760
- WO-A-96/20215
- WO-A-96/40941
- WO-A-97/02839
- WO-A-98/13066
- WO-A-03/004049
- RIECKMANN P: "IMMUNOMODULATORY THERAPY OF MULTIPLE SCLEROSIS: CURRENT TRIALS AND FUTURE PERSPECTIVES" MOLECULAR SIGNALING AND REGULATION IN GLIAL CELLS. A KEY TO REMYELINATION AND FUNCTIONAL REPAIR, XX, XX, 1997, pages 312-321, XP001056478
- WEINER H L ET AL: "THERAPY FOR MULTIPLE SCLEROSIS" NEUROLOGIC CLINICS, XX, XX, vol. 13, no. 1, February 1995 (1995-02), pages 173-196, XP001121805
- POLMAN C H: "OTHER IMMUNOMODULATORY THERAPIES IN MULTIPLE SCLEROSIS" BAILLIERE'S CLINICAL NEUROLOGY, LONDON, GB, vol. 6, no. 3, 1997, pages 511-524, XP001006179 ISSN: 0961-0421
- ARNASON B G W: "IMMUNOLOGIC THERAPY OF MULTIPLE SCLEROSIS" ANNUAL REVIEW OF MEDICINE: SELECTED TOPICS IN THE CLINICAL SCIENCES, ANNUAL REVIEWS INC., PALO ALTO, CA, US, vol. 50, 1999, pages 291-302, XP000885669 ISSN: 0066-4219
- SAIFUDDIN M ET AL: "Expression of MHC class II in T cells is associated with increased HIV-1 expression." CLINICAL AND EXPERIMENTAL IMMUNOLOGY, vol. 121, no. 2, August 2000 (2000-08), pages 324-331, XP002227061 ISSN: 0009-9104
- DATABASE WPI Section Ch, Week 199044 Derwent Publications Ltd., London, GB; Class B04, AN 1990-331416 XP002227065 & JP 02 237935 A (BIO KAGAKU KENKYUSH), 20 September 1990 (1990-09-20)
- JONES-BRANDO LORRAINE V ET AL: "Metabolites of the antipsychotic agent clozapine inhibit the replication of human immunodeficiency virus type 1." SCHIZOPHRENIA RESEARCH, vol. 25, no. 1, 1997, pages 63-70, XP002227062 ISSN: 0920-9964
- KUTSCH O ET AL: "Induction of the chemokines interleukin-8 and IP-10 by human immunodeficiency virus type 1 Tat in astrocytes." JOURNAL OF VIROLOGY, vol. 74, no. 19, October 2000 (2000-10), pages 9214-9221, XP002227063 ISSN: 0022-538X
- SILVESTRIS FRANCO ET AL: "Autoreactivity in HIV-1 infection: The role of molecular mimicry." CLINICAL IMMUNOLOGY AND IMMUNOPATHOLOGY, vol. 75, no. 3, 1995, pages 197-205, XP002227064 ISSN: 0090-1229
- MACKENZIE R. ET AL: 'Hyperimmune goat serum for amyotrophic lateral sclerosis' JOURNAL OF CLINICAL NEUROSCIENCE 2006,
- INTERNET CITATION, [Online] Retrieved from the Internet: <URL:http://www.nlm.nih.gov/cgi/mesh/2K/MB_ cgi?term=Motor+Neuron+Disease&field=entry> [retrieved on 2009-11-12]

## Description

### BACKGROUND OF THE INVENTION

WO 97/02839 relates to Viral Suppression, Treatment and Prevention of Viral Infections. It provides a method for producing neutralizing antibodies for the treatment of a viral infection in a patient, comprising the steps of:
a. exposing a mammal to a virus such that said mammal produces neutralizing antibodies to said virus and
b. collecting said neutralizing antibodies from said mammal.

In the examples, an HIV vaccine designated AAV₂ is obtained by mixing HIV virus with HIV neutralizing antibodies obtained from a goat.

WO 01/60156 relates to Neutralizing Antibody and Immunomodulatory Enhancing Compositions. It provides an immunomodulatory composition comprising:
heterologous antibodies specific for an antigen; and
an antigen, wherein the heterologous antibodies form a complex with the antigen for combination with a pharmaceutical carrier.
The examples are similar to those of WO 97/02839, and again an HIV vaccine designated AAV2 is obtained by mixing HIV virus with HIV neutralizing antibodies obtained from a goat.

WO 02/07760 is concerned with a Therapeutic Agent. It provides a method of preventing HIV infection or treating an individual infected with HIV, comprising the steps of
(1) exposure of goat immune system to HIV;
(2) purification of antibody from the goat after HIV challenge; and
(3) treatment of an individual with the antibody obtained in step 2 above.

WO 03/004049 is concerned with a therapeutic agent, and reports on the discovery of anti-HLA and other antibody activity in a composition prepared from goat serum following HIV challenge.

In preliminary trials based on the product of WO03/004049, patients with HIV have been treated successfully using serum from a goat after challenge with HIV.

Preferably the treatment employs a serum composition which can be obtained by a process involving raising effective antibodies in a goat, draining blood from the goat, demonstrating HIV neutralising capability in the drawn blood, removing solids from the blood, precipitating using supersaturated ammonium sulphate or other suitable precipitation agent, separating the precipitate, dissolving the precipitate in a suitable aqueous medium, and dialysing the solution with a cut-off of 5,000 to 50,000 Daltons, preferably 7,000 to 30,000 Daltons, more preferably 8,500 to 15,000 Daltons, especially about 10,000 Daltons. The method of goat immunisation can be intramuscular but other standard techniques such as subcutaneous or intradermal adminstration can also be used. The purification process can also be completed by other commonly used fractionation action methods (caprylic acid for example) provided the total residue is used.

More particularly, the treatment typically employs a goat serum obtained in the following way.

### Example of Production of Goat Serum

A goat was inoculated by intramuscular injection with lysed HIV-3b virus suspended in a normal commercial supernate, using an intra-muscular injection of HIV-3b at a concentration of 10⁹ viral particles per ml. The virus was previously heat killed at 60°C for 30 minutes. Blood samples were drawn after an appropriate interval, such as two weeks, for initial assessment. In the optimised procedure, the goat is injected every week for four weeks, then at six weeks the animal is then bled to obtain the reagent.

Approximately 400 cc of blood is drawn from the goat under sterile technique. The area for needle extraction is shaved and prepared with betadine. An 18-gage needle is used to draw approximately 400 cc of blood from the animal. Of note is that the animal can tolerate approximately 400 cc of blood drawn without the animal suffering any untoward effects. The animal does not have to be sacrificed. The animal can then be re-bled in approximately 10 to 14 days after it replenishes its blood volume.

The presence of potentially useful antibodies was confirmed. Once the presence of such reagents was confirmed blood was then taken from the goat at between 4-6 weeks, and centrifuged to separate the serum. 300ml of serum was then filtered to remove large clots and particulate matter. The serum was then treated with supersaturated ammonium sulfate (45% solution at room temperature) to precipitate antibodies and other material. The resulting solution was centrifuged at 5000 rpm for five minutes, after which the supernatant fluid was removed. The precipitated immunoglobulin was resuspended in phosphate-buffered saline ('PBS buffer', see Sambrook et. al. 'Molecular cloning, A Laboratory Manual', 1989) sufficient to redissolve the precipitate.

The solution was then dialysed through a membrane with a molecular weight cut off of 10,000 Daltons. Dialysis was carried out in PBS buffer, changed every four hours over a period of 24 hours. Dialysis was carried out at 4°C.

After 24 hours of dialysis the contents of the dialysis bag were emptied into a sterile beaker. The solution was adjusted such that the mass per unit volume = 10 mg per ml. The dilution was carried out using PBS. The resulting solution was then filtered through a 0.2 micron filter into a sterile container. After filtration, the solution was divided into aliquots to give single doses of 1ml and stored at -22°C prior to use.

The reagent is then ready for use.

Changes may be made in this procedure, such as for example by varying the concentration of the ammonium sulphate or switching to ther reagents. Similarly the dialysis cut-off need not be at 10,000 Daltons.

### THE INVENTION

The present invention provides use of a serum composition obtained from a goat after challenge with HIV, in accordance with claim 1 or claim 2.

In general, administration of the composition to a human can result in a significant improvement in one or more of these conditions. One or more administrations may be given, and typically the benefits are observed after a series of at least three, five or more administrations.

Typically the invention employs a composition including the active component which can be derived from the blood of a suitably challenged goat by a serum extraction technique that is not designed to isolate individual, specific antibodies. In particular, the invention envisages isolation of the active component, which we currently believe includes anti-HLA antibody from blood serum of the challenged goat, without the requirement for exhaustive purification and extraction to obtain an individual antibody.

Without being bound by any theory or hypothesis, it seems that the working substance may include a biologically active goat molecule that is not a goat antibody, but a cytokine, hormone, or similar type molecule that is retained through the crude purification process.

If the active substance is an antibody then it may be recognising one or more ligands or receptors which in vivo are triggering the physiological effects we are observing.

In general, injection of antibodies or serum compositions into humans derived from a non-human host is counter-indicated. A strong immune response is normally mounted against the foreign antibodies themselves. However, surprisingly, it has been discovered that use of goat serum extract does not provoke the immune reactions which are anticipated with other foreign animal proteins. Injection of goat serum extract is tolerated both by immunosuppressed patients and normal individuals.

The present invention specifically uses a serum extract, which possibly comprises the total population of antibody molecules including anti-HLA activity, derived from HIV challenge to a goat. Without wishing to be constrained by theory, we believe that such an approach possesses significant benefits. Patients treated with such a serum extract showed significant effects within minutes of being treated.

A killed virus is injected into a specifically identified goat, by intramuscular injection, and allowed to incubate. Thereafter a measured quantity of blood is drawn and modified accordingly.

After inoculation of a selected goat with the HIV virus, an immune response due to exposure to a foreign protein antigen, was noted in accordance with earlier studies. The extracted serum was then further modified in order to prepare it for human use.

Treatment is given by means of a subcutaneous injection, in amounts varying between one/tenth and ten cc and is designed to deliver the medication as speedily as possible to the lymphatic system. With the present invention, the preferred dose is usually 1 ml weekly or as required, given as a divided dose into both arms. Administration every 2 or 3 weeks becomes typical, then every 3 months.

In most cases the treatment has been conducted once ever four weeks over a three month period. General observations are as follows:
1. Moderate to severe depression was reversed in less than sixty minutes post injection.
2. Patients generally within two hours post injection regained their appetites and actively sought out food.
3. Within approximately two weeks of the first treatment the patients started to gain weight.
4. Independent laboratory reports confirmed that 4 to 6 weeks after the first treatment the viral loads and P24 values were dropping substantially and that CD4 and CD8 cells were increasing dramatically.
5. Significant side effects were not observed.

It is important to note that the present medication, unlike current treatments, does not require the patient to maintain a strict hourly or daily regime and relies upon a simple injection being administered either weekly or monthly.

Preferably, the composition is purified and consists essentially only of a purified serum extract. In a further variation, the product may also be purified by conventional or any other suitable procedure, including, but not limited to, for example immunaffinity chromatography, salt precipitation, ion exchange chromatogrpahy, size chromatography, affinity chromatography, in combination as appropriate or desired.

A test dose is employed usually to see if the person develops an allergic reaction to the hyperimmune goat serum. An intradermal injection is followed by a wait of 30 minutes to see if there is an intermediate reaction which is manifested as oedema, erythemia, and itching. If this reaction is negative, then the assumption is that an immediate sensitivity reaction is most likely to occur. An allergic reaction does not preclude the person, however, from receiving a potential life-saving treatment because of a possible allergic reaction.

Administration of the composition of the invention may be by any suitable method such as by intravenous infusion, subcutaneously, intramuscular injection, oral preparation, intraperitoneal and intravenous administration. The correct dosage will vary according to the particular formulation, the mode of application, and the particular situs, host and condition being treated. Other factors like age, body weight, sex, diet, time of administration, rate of excretion, condition of the host, drug combinations, reaction sensitivity and disease severity shall be taken into account. Administration can be carried out continuously or periodically within the maximum tolerated dose.

Unlike existing drugs, which often need to be taken daily for the rest of the patients life, a typical treatment relies upon a simple injection being administered by a doctor either weekly or monthly. A normal treatment programme is of three months duration, with an anticipated follow up procedure at six months, twelve months and two years or as necessary should the virus reappear.

The composition of the present invention may be used with other drugs to provide a combination therapy. The other drugs may form part of the same composition, or be provided as a separate composition for administration at the same time.

Without being bound by any theory, we believe that the presence of anti-HLA activity is an important constituent of the product of this invention. Preferably any antibody in the product of this invention is a polyclonal antibody that recognises a repertoire of HLA class II antigen and gp 120 antigen Our findings suggests that it is preferable to have HLA class II antigen. Notwithstanding our preference for anti-HLA activity, we do not eliminate the possibility that other active active molecules are present. In this respect, the compositions of this invention may therefore include an additional bioactive compound. We have already demonstrated that oral administration gives similar effects as injections. IgG should not cross into the blood stream from the mouth, which lead one to believe that IgG may not be the only active component.

Suitable products can be raised by employing as immunogen a selection of antigens, preferably a cocktail of antigens. It is possible that the use of a range of different antigens give rise to antibody which recognises common structures of the antigens giving a stronger response in the patient. We hypothesise that a selection of HIV isolates will provide epitopes with minor variations in structure, and a pan-antibody will result.

Thus, to generate the serum, we prefer to employ a cocktail of different HIV viruses produced primarily in PBMCs, rather than use T-cells alone. The cocktail suitably contains 2, 3, 4, 5, 6 or more of such viruses. The viruses are preferably in the form of lysates, since lysates are non-infectious and serve to expose the immune system to internal proteins. Examples of preferred lysates include the following HIV-1 isolates: 91US056, 92HT593, 92US723, 92US657, 92US660 and 92US714. Preferably the cocktail includes at least 1, 2, 3, 4, 5 or all 6 of these particular isolates.

Thus, cocktails of similar cells can be employed (multiple PBMC donors, Multiple T Cell donors, Multiple activated Nerve cell donors). Multiple cell sources are expected to broaden the cellular protein range. This broadening might help alleviate differences in improvements one might expect with patients with different HLA types etc.

Such preparations can also be obtained using proteins containing the peptides isolated from HIV lysates, synthetic peptides, synthetic oligopeptides. Antibodies to lysate can be obtained and tested.

Without being bound by our current theory, it seems that the antibody acts to suppress cell proliferation of the kind which is required by HIV or other conditions reliant on such an immune response. Thus, for example, the present invention finds application in the treatment of multiple sclerosis.

As well as palliative treatments, the antibody product is of use for the treatment of diseases with an inflammatory component, and includes not only HIV, but also neuritis.

We have studied sera from different goats immunised with different - virus/cell preparations and can show that cocktail injected goats produced an immune response which sees many of the cryptic i.e. silent parts of the HIV envelope, which may well be important.

The sera seems to be particularly suitable for diseases associated with chronically activated cells which may be secreting damaging messengers such as cytokines and chemokines. These include multiple sclerosis, all forms of chronic inflammatory conditions of the nervous system.

In a particular aspect of this invention, the composition of this invention is employed both to provide palliative improvement in the condition of a patient and to treat a disease such as multiple sclerosis.

The recovery seen in many of the MS patients, alongside the elevated mood reported within the hour of receiving the treatment, has also prompted us to look for activity against receptors in the CNS which may be involved in nerve stimulation and possible regeneration. We have screened the various sera for activity against a number of antigens and have found activity against the dopamine receptor, serotonin receptor, Nerve growth factor receptor p75 and the chemokine CXCL10(IP10).

The combination of anti-FAS and/or anti-HLA antibodies may be important, along with antibody against one or more of dopamine receptor, serotonin receptor, Nerve growth factor receptor p75 or chemokine CXCL10 and thus assays might be directed at the various antibody activities to ensure their presence in the product.

The anti class 2 polyclonal antibodies in this sera may work not only directly but indirectly. By the direct approach they would block the inappropriate presentation of peptides leading to the auto immune inflammatory destructive process.

The further mechanism is that by binding to class 2 on cells which would normally express the class 2 this may induce cell death of apoptosis. This is the same type of pathway which would be induced via the fas L network.

It has been reported that how cells die i.e.; by apoptosis or necrosis is crucially important as to the immune response to the antigens on the cells. In MS cell mediated immunity appears to be dominant over humeral immunity and treatments aimed at pushing the humeral or TH2 type of immune response are thought to be desirable in multiple-sclerosis. In the few patients we have examined for this activity before and after the administration of the serum they do indeed switch to the desired immune response. The desired immune response includes the inductional cytokines IL4, IL6, IL10.

Recent animal data suggests that animal models of MS can be improved with the induction of re-myelination using cytokines of the IL6 family which are part of the TH2 humeral path way.

The following points arise:
1. There is a strong position to claim that the inhibition of MHC class 2 polyclonal antibodies is probably able to induce more than one activity i.e. in as mentioned above one locking in appropriate presentation of self peptides and hence blocking the paracrine drive which results in the demyelination. (Paracrine meeting inducing responses from other cells which feed the damage).
2. Attaching to class 2 and inducing apoptosis of the cells either directly or by inducing antibody directed cytotoxicity of which there are multiple mechanisms.
3. By causing the cell death of the inflamed cells in such a way that they are taken up by antigen presenting cells and presented to the immune system in such a way that it shifts the immune bias to a TH 2 response which include the induction of IL6 responses which lead to the re-myelination of the de-myelenated plaques which are the hallmark of MS disease.4. The ability of class 2 to induce apoptosis on cell lines with inappropriate class 2 on mean that this treatment could be induced for those rare tumour types that hyper express MAC class 2. It has recently been shown that antibodies against these cell lines will induce apoptosis. We have noted increased apoptosis in some of our cell lines.
4. Several lymphomas and late stage melanomas express high levels of HLA class 2. Hence the polyclonal sera could be better treatment for these tumours than monoclonal therapies which are currently in development.

Several recent publications have emphasised the role of cytokines in driving the damage in MS. These include IL 1 and TGF beta which is alleged to increase a molecule called jagged 1 which appears to high in MS plaques and down regulates in re-myleninating lesions. 2. There is also considerable evidence that inflammatory cells exhibit resistance to apoptosis with higher expression of BCL2 and aflip proteins and that these are not affected by treatment with interferon beta. Induction of apoptotic pathways to circumvent the protective BCL2 pathway maybe an important component in the activity of the serum.
5. There is another route which has recently become more recognised as having potential importance and that is the claimed role for MHC molecules (HLA in human) in the development of the nervous system. This works which at first appears to defy both immunological and neurological dogma shows how MHC molecules present on brain tissue determine how the brain tissue interacts with its environment and regulates neural development especially of the eye. Inappropriate HLA expression when locked may explain the rapid re-organisation and re-myelination seen in some of these patients who have had visual improvement following extant visual impairment of several years standing.
6. These observations include those previously made for HIV which is nonspecific and could be used for the treatment of any chronic infectious disease. This can probably be the down-regulation of the chronic inflammatory processes.
7. A dramatic improvement in severe gout has been noted following the administration of serum. A dramatic improvement in wellness and increase in energy levels have been noted by most of these patients.
8. An improvement in two patients with Parkinson's disease. This may either be due to the induction of the necessary transmission factors or maybe due to its anti-inflammatory effect on an agent driving the disease. (chronic helicobater infection has been postulated).
9. There has been a marked effect on patients with carototic lesions and both early squamous cell carcinomas and basal cell carcinomas have been resolved on this treatment. This is extremely unlikely to ever happen without active treatment. It therefore transpires the serum acts directly or indirectly by inducing various cytokines/chemochines etc. which lead to the induction of apoptosis and resolution of these skin lesions.
10. The induction of a nerve growth factor re-myelinating factor is important in its own right as two patients have had regeneration of traumatic nerve severation nothing to do with their MS. This is encompassed scientifically again either by direct activity or the indirect induction of the afore said growth factors including IL6. Although it is possible to explain many of the features above by either direct or indirect responses to the administration goat serum with anti-DR antibodies (and the DR aspect is probably extremely important as opposed to other class 2 types in general as these inhibit the next lymphocyte responses which the goat inoculating goat serum does incredibly well, with other anti class 2 antibody responses not being nearly so efficient).

It is also possible that there is another very important agent apart for other antibodies, and known anti inflammatory targets previously mentioned including fassil and Icam.

Beneficial effects may well be caused by a molecule or antibody induced by the inoculation vaccination process.

The fact that there must be some such antibody is rendered more likely by the fact that all patients experience a feeling of extreme well being within 15 mins of the first injection.

A cardinal feature of MS lesions is the inappropriate expression of HLA-DR on neuronal cells including astrocytes (Traugott and Lebon, J.Neurol 1988 April 84 257 and Microglia J.Neurol Science 1987 August 80 25-37). It is thought that this inappropriate expression presents self-neuronal antigens to the immune system and also acts as a target. Cells expressing inappropriate Class II are associated with a classic ongoing inflammatory response. Anti-inflammatory treatment per se is well known to dramatically improve the relapsing remitting type of MS. More recently it has been shown that the resulting products of inflammation such as cytokine and chemokines may be responsible for the neuronal damage and demyelination in particular. In a recent mouse virus model of a chronic demyelinating disease it was shown that neutralisation of the chemokine CXCL10 reduced the inflammatory cell invasion demeylination and actually improved neurological function. The model showed a pronounced suppression of ongoing demeylination which was associated with improved neurological function. This was associated with marked remyelination. Thus observations in both classic laboratory models as well as our own suggest that the damage seen in multiple sclerosis may not be as acute and permanent as the clinical features of secondary progressive MS suggest and that the chronic inflammatory state is akin to shorting out the neurological system as opposed to permanently damaging it, at least in the early phases.

The inflammatory cascade is associated with high gamma interferon levels which are thought to lead to the inappropriate up regulating of HLA Class II on neurological tissue as well as marked inflammatory response leading to multiple cytokine and chemokine affects. Further studies on this sera have shown that it has activity against at least 3 different inflammatory pathways. Moreover, all patients treated to date who have been able to provide sera before and after treatment have shown a reduction in gamma interferon and an increase in TH2 cytokine production which is the desired immunological effect of an ideal immunotherapy.

There is yet another pathway that needs to be considered, and that is the downstream immunomodulatory effects evoked by triggering certain HLA class II molecules and inter-related complexes on the surface of certain APC populations. Thus, apart from physical loss of the offending APCs by apoptotic removal we may actually be triggering anti-inflammatory cytokines and regeneratory growth and repair factors.

The goats are likely producing a factor on the surface of a human lymphoma cell line which has changed its phenotype when infected with this deceptive foe (HIV). Indeed, in addition to induction of xeno-type responses in goats to everything foreign on the human lymphoma cells, these cells have also unregulated certain molecules (danger-like ligands) in response to presence of HIV in these cells.

We treat patients very successfully with vision problems when they occur as part of the general debility problems associated with multiple sclerosis. Not all patients get this problem, but in those that do, it usually disappears within a few days, (i.e.) they get their vision back.

The same type of visual problem seems also to occur in patients who do not have MS.

In the opening public lecture of the 32^{nd} annual meeting of the Society for Neuroscience on how histocompatibility builds the brain Investigator, Carla Shatz not only described how the visual system is established, she discussed her latest revelation: Molecules best known in immune recognition are involved in building the neural connection system.

Many scientists liken neural connections in the brain to their electrical counterparts in a computer. But Shatz, who specializes at Harvard Medical School in the development of mammalian visual systems, showed that connections in the mammalian brain are much more flexible than the hardwired links in computer chips.

During eye development, signals are relayed from the retina to the lateral geniculate nucleus (LGN), and then on to the visual cortex of the brain, where the information is processed. Initially the connections from one eye are randomly intermixed with those from the other eye in the LGN and at the cortex. But early in development - before the eye even opens or functions - neighbouring groups of retinal neurons fire in waves, without any input signal. These waves of autoimpluses from the neighbouring retinal neurons seem to strengthen some connections in the LGN and cortex, while weakening others.

The end result is that the signals from one eye are interspersed in a layered pattern with inputs from the other. Like the black and white stripes of a zebra, the stripes of input from each eye are juxtaposed, but not intermixed, in the mature brain.

Shatz's group has shown previously that if they block the autoimpulses from the retina to the LGN, then the neat layering of the normal adult pattern fails to form, and the immature intermixed pattern remains. Thus the researchers conclude that the adult pattern is the result of simultaneous weakening of some connections and reinforcement of others. But the exact molecules involved in the process have remained largely unknown.

Now Shatz and colleagues have made the surprising discovery that proteins in the major histocompatibility complex class I (MHCI) are directly involved in the process. Previously, MHC molecules were thought to be in the sole purview of the immune system, where they are responsible for presenting foreign antigens to E-cells and inducing cellular immune response. But when Shatz's group used microarrays to compare mRNA from LGN neurons that had normal inputs from the retina to ones that had the connections blocked, they found the MHCI RNA was present only in the normal LGN.

Shatz's group also has light microscope data indicating that the MHCI proteins are present at the synapse in several regions of both the developing brain and the adult brain.

Her current hypothesis is that the MHCI proteins are the "anti-glue" that allows the inappropriate early connections to be broken down. Imagine the MHC1 protein sitting on the in the membrane of the post-synaptic neuron, she suggests, and interacting with a protein complex on the presynaptic neuron. Shatz speculates that the interaction initiates a signalling cascade that somehow reduces the strength of the connection, eventually leading to its complete elimination.

### COMPARATIVE EXAMPLES

### Treatment of Disease

Patients (who are under self-administered treatment and/or supervision from their doctor and/or treatment overseas) have taken the treatment by subcutaneous injection. The number of patients is shown in brackets.

The conditions they are suffering from, and the observations that have been made, are as follows:-
1. Cancer and Growths (8/9).
   For terminally ill patients, increased life beyond the prognosis given by their hospital or doctor.
   Melanomas and certain other skin pre-cancers disappear. Sebaceous and Other Cysts. They disappear.
   Reduction in fibroids (female)
   Patient AAA (male). A former PT instructor in the Royal Marines with throat cancer (one effect of which was impaired speech). He is now free from this condition. He has resumed his daily fitness programme (particularly bench press-ups) more actively than when he was in the Marines.
   Patient BBB (female). With uterine polyps. Now free of this condition.
   Patient CCC (male). Rodent ulcer/weeping sore. Now free of this condition.
2. AIDS/HIV (100+)
   Maintained health levels. Absence of secondary infections (colds, thrush etc.)
3. Multiple Sclerosis (30)
   This is the most closely observed group and, apart from the AIDS/HIV patients, it is the largest in number. All have felt the improvements in condition listed in (A) above. There is increased mobility, improved co-ordination and an improved ability to 'look after oneself', with less dependence on care assistances.
   Patients whose deteriorated condition had led to impaired bladder function and bowel movements, report a significant restoration of normal functioning to bladder and bowel.
4. Motor Neurone disease/ALS/Muscular Dystrophy (1)
   Improved mobility and co-ordination.
5. Gout (1-3).
   There is one principal patient, but two other patients being treated for other conditions have reported positively on their previous intermittent gout attacks.
6. Parkinson's Disease (3)
   Improved co-ordination.
7. Rheumatoid Arthritis and other Bone/Joint Ailments (1)
   The patient reports no recurrent attacks of rheumatoid arthritis and an absence of inflammation.
   In other patients there is evidence of reduction in osteoarthritic nodes and, in the case of osteoporosis, increased bone density.
8. Bone and Nerve Re-Growth (2)
   Observational evidence seems to imply some re-growth, repair, or partial re-connection for two patients.
   Patient DDD Lamenectomy. Severe damage to 1 vertebra. Used to walk with sticks and needed a wheel-chair to board aircraft. Had pain and movement was limited. Now mobile and pain-free
   Patient EEE An MS sufferer. Has observed return of 'feeling' in nose and a damaged finger.
   A third patient's condition of stenosis was relieved.
9. Eye Sight /Neural Connection Systems
   The treatment appears to have been effective in removing cataracts in two patients. A number of MS patients with retinal atrophy have experienced significant restoration of eyesight which was either failing or, in the case of one patient, which had been blindness over number of years.
10. Diabetes
   A patient with Type 2 diabetes has reported that their blood sugar levels have returned to normal.
11. Inflammatory Relief
   The treatment appears to give relief for a number of inflammatory conditions. This is a common observation in the broad range of conditions described above, and in the specific illnesses described below. There is no collected data for asthma (being inflammation of the lung tissue) but one patient may be showing some relief of this condition. A number of heavy cigarette smokers may also be experiencing easier breathing.

### Examples of Comments from Patients

### Patient ZZ

After having MS for 27 years this November I felt there would be a cure on the horizon but I felt I would not see it in my lifetime. I just thought that I would see it but only for a few new babies that would be vaccinated against it like Polio etc. that was my feelings anyway. Last October when some friends phoned me and told me they were able to get me this programme I was thrilled, I thought my prayers had been answered.

Even after the very first injection when I had amazing results my eyesight had begun to improve after a few minutes, and the doctor and another person who were sitting in the surgery with myself and my husband thought it was amazing too.

After that I went from strength to strength, my walking was fantastic and around the house I didn't have to use my stick or in the few weeks previous a walking frame that the hospital had loaned me. My wheelchair had become redundant expect when we went for longer walks.

Even after my husband and I had caught one of those nasty viruses which knocked us for six, in fact he even passed out and hit his head on the radiator which left a nasty bruise on his forehead, I was just unable to walk anywhere without help of some sort. This all happened last January and other than felling weak for a while I picked up very quickly. I do fell though that if it wasn't for this treatment I would have had a relapse and been very poorly and maybe even sent to hospital and been on steroids just like I had in the past after things like this happened to me.

As time went on we did have a couple of blips, but nothing I couldn't cope with as I knew what this treatment could do for me in the future. I have even been asked to talk on the phone to others who are considering starting this treatment and I am still in contact with them now, so we know how we are doing and once again when they maybe having blips I can tell them not to worry as it turns out OK and to stay with it. I can honestly say hand on heart that this treatment has given me back a marvellous quality of life that I thought I had lost, it is truly the light at the end of the tunnel come true for me and for many other and I could go on and on about it but there is not enough hours in a day to do it justice as far as I'm concerned. Thank you for finding time to read this.

### Patient YY

Since taking medication, small, but clear improvements have occurred:
- The right eye, which up to then had been static, moves again
- Bladder weakness has almost gone
- I feel I have more energy, and tiredness during the day has decreased
- Balance has become much better, but sill unsteady
- I can stand up straight again and do not rock
- I can use both legs again when going upstairs; always going up, and often when coming down.

There is no improvement in style when walking, but there is an improvement in duration; distances are slowly getting longer.

Note: I have only been taking medicine for a short period: the first injection was made 11 weeks ago.

### Patient XX

I have been using the new animal serum since 21/03/02. I have been diagnosed with MS and the only therapy available to me up to now is Avonex Interferon beta-1a. The Interferon certainly has no visible effects on my condition which has progressed to difficulty in the gait, a stiffness in my legs, problems with urgency and frequency of urination and difficulties with the bowel.

From my very first application of this serum, I discarded my stick which I very rarely us now, the problems with the urgency and frequency have all but disappeared and although my gait has not noticeably improved my general sprits have because I genuinely feel that it has made a great and positive difference in my symptomatic picture. I am a firm believer in this line of approach to this disagreeable illness and I urge all concerned people to please take note of its helpful and dramatic effects.

### Patient WW

Between December 2000 and 2002 I have been in hospital and rehabilitation 6 times due to MS attacks, my problems were dizziness, loss of balance, tingles in my feet, legs hands and back. Patchy numbness in my feet, legs, arm and face, stiffness in my legs, twitching inside the right hand side of my face and in both eyes. A pulling sensation in my head and not being able to walk properly, sometimes my walking was reduced to just a few steps, I had no energy and suffered very badly from depression, was always sleeping and had no interest in anything - I had nothing positive to hold onto. These are just the problems I have had since December 2000.

June 19^{th} 2002 changed my life around, I had my first injection of the serum, since then I have never looked back. The tingles and the patchy numbness is reduced, I still have the tingles in my right foot but not at all like it used to be. My balance is much better and I have so much energy now and I don't sleep like I use to.

My right pupil which has dilated since the diagnosis of Optic Neuritis in 1986 is now the same size as the left pupil. 6 weeks ago I actually made my son's bed and I'm starting to do small jobs around my flat. I went shopping 2 weeks ago, it was the first time in may months and I stood in the supermarket for nearly 2 hours, I haven't done that for nearly 2 years. I now go swimming which wasn't possible before due to the weakness in my legs, and I meet regularly with friends, I still have my bad days but they are becoming fewer as the weeks go by.

Since I have been taking the serum it has given me back my life, dignity and self esteem and my children back their mother.

My physiotherapist has said my legs are approx 15% stronger and that's after just 6 weeks.

### Patient W

I am 44 years old. I am married and have three wonderful children.

I was officially diagnosed with multiple sclerosis three years ago. I say officially as along with most fellow sufferers, the realisation of one's symptoms and condition brings with it the realisation that indeed these symptoms were preset for quite a while but just thought that you had been dumsy and under the weather.

In fact my symptoms were classed as many weird and wonderful thins including the menopause. I cannot put all the blame on those who should know as the information regarding this illness and the lack of things to treat it with cause problems. I did try intaferon, it didn't work for me.

Anyway by the time I had been diagnosed things started to progress. Two years ago I was at the point of despair. Spasms in my legs, I dragged my right leg and would pull it along using my left hand, as my right hand was getting unusable. I dropped everything I tried to grab or reach for. I became increasingly disorientated falling over all the time if I was unaided. My memory was not functioning properly in mid flow of conversation the words would leave me, frustrating and embarrassing. I had to be reminded of the most simplistic things, and worst of all I had misplaced my memories of precious gems, one being those of my children's baby day's and most of their growing years. My speech then followed and I have periods of not being able to communicate properly.

My appetite diminished and sleep although badly needed as exhaustion was incredible and I had no choice but to take to bed ruing the day sometimes nearly all day, was sometimes a nightmare because the spasms would wake me up, and so too my husband in the night.

My eyesight had begun to give problems, I had previously never suffered in this way. My bladder was very badly effected and so too my bowels. Life had changed dramatically not for good; I became and was beginning to take on the life of a recluse because to attempt to go out was a nightmare. If it was a public place ie, a restaurant, cinema etc there would have to be a sweep of the intended destination to see where the toilets were, if it had steps in the building to negotiate, how many and what facilities were there for people like me, and believe me there weren't many, also that at a moment notice I might have to cancel because I was not strong enough to attend.

Life was bad and for my family as well because they were victim too of this beast within. I couldn't participate in the normal parent children things, and the children were worried sick. We had no future and couldn't plan one because I was spiralling downwards. It affected our marriage and I began to realise that it could possible that I wouldn't be around for my children, that really hurt. Also the fact that I was not going to get better because there was nothing that could help, and so faced with this, started planning my way to eventually ending up in residential care my choice, as I had lost myself and making choices in life, the beast was taking over and my last and only choice would be one of dignity.

Then I came across this Gift of Life, and embarked upon the next journey of life which to be honest when told about it thought well I have nothing to lose and it didn't take me long to agree to try.

This came to be the best decision ever, I started receiving the injections and could arise out of a chair from sitting position straightway rather shakily, impossible usually. In fact I basically crawled in and came out waling. I was thrilled, and wept with tears of complete joy. I continued the injections and every week a new revelation within my body and the restoration of things that had been torn away from me.

And now two years later I can walk perfectly well, my speech is fine my co-ordination is great, my bladder I have no problems with so too my bowels, I eat well and my memory has begun to filter back both short term, and long term. Well I am reclaiming my treasured gems and am beginning to have a mental full diary of my children's past

My stamina is now very good and so are my energy levels. I in fact look after my parents my father being rendered disable 1 year ago, my mother does not keep the best of health either so I run tow households now and find time for some voluntary work.

See without this wonderful medicine I dread to think where I would have been now, if here at all. I have been given my life back, and so have my family. The children have been given their mother back to them and my husband has now got his wife back. We can and do plan our future as now even feel that now we do have one. I never dreamt in a million years that this was possible but it is and I'm living proof. I have been given the Gift of Life.

Fairy tales do come true and we will live happily ever after.

### Patient UU

Please find some observations I have noticed with only modes use of the 1gG. For the record, the most I have ever taken is 2.5 mg on a 4 week cycle.

Concrete items that I can give specific details include:
First and foremost the carototic lesions below my eyes are under control. Aside from scientific curiosity, this was the prime personal reason for trying the IgG. I spent the better part of a decade in an outside environment with much exposure to the sun. As a result, I have had significant scaling and redness below both eyes for a number of years. Creams and lotions would temper the situation, but it was always red. At the same time, my facial skin is supple. Body skin does not feel as "dry" as it should for this time of the year. Lotion is not required on my shoulders on a daily basis as before. I currently still apply lotions to the skin, but not significantly different than I have for the past four or five years.

I have not had a significant cold in over a year despite - 12 trips to the UK from the USA, and coming home to kids in school. This may be just good luck, or good living, but I would have expected a real "knock down" type of germ. Please note that I have had a number of "near misses", but no direct hits.

I have not needed coal tar based shampoo to control dandruff in months. Usually, this would be a daily requirement, especially in the months between October-April. Even with the coal tar based shampoo treatment, I would have an itchy scalp within 24, hours. Now I am using a milder shampoo this November. Furthermore, if I miss a day, I do not feel the "itch" like I would have previously.

There is more prostate fluid being manufactured. Speaking of the urinary area, I have noticed that there are fewer "lingering" drips after urinations. The speed of shutting off the urine stream is much closer to what it was when I was 20-30. I noticed this shutting off process slowed down about 6-7 years ago.

I find there is no more morning "stiffness". There is no minute or two needed to "limber up".

Less concrete observations:
I notice that I just seems to have more "energy" and more alert. This is not like having an extra cup or two coffee; it just seems that I can concentrate better.

There is also a general feeling of "well being". This is not like being intoxicated. It is more like how once feels after a "really" good night sleep.

A final area where I notice improvement, but have problems documenting a "before and after" is my muscle tone. I seem to be able to retain muscle tone longer. For example, I did not run in the morning during my last trip to the UK. I normally get out at lest 2 mornings for a run... This gave me a 10 day lag between morning runs. Upon my return, I felt no stain on my first run and actually got a "runners high". The runners high should have taken at least one or two more AM runs to attain again.

My eight year old Border collie (sheepdog) Taran has hip displasia and currently takes 150mg rimadly to relieve discomfort in his hips. Being a working border collie that I personally trained, we have an understanding of each other that is much more intense than a traditional dog/human relation. In order for us to work the animals together, we both need to know exactly what the other is thinking. On Wednesday, Nov. 19, he received 15 mg orally of the 1gG. I could tell that he got the immediate "feeling" of well being as he stayed in my office next to my chair for an hour or so with a slightly different expression. Three days later, I notice that he is twisting his upper body and spinning much more when playing with our younger dog. He is also following me around the house much more as opposed to lying on his heated mattress. His facial expressions were that of a younger and "happier dog".

I am unable to tell if it is actually helping his hip significantly, but is seems to be helping with other aliments like stiffness which I was unaware he was feeling. There also seemed to be a general feeling of well being. I plan to give another 1.5 mg this upcoming week to see if my initial impressions continue.

### Further Patients

A patient who had suffered from progressive multiple sclerosis for 3 years volunteered to try the serum. In short, she had a marked clinical improvement which is maintained over 6 months since commencing the treatment. Objective improvement was reported in at least two other patients. As fellow MS sufferers learnt of the improvement they requested access to treatment. Prior to treatment it was recommended that a formal functional assessment should be carried out by neurologists and that this be repeated one month later. Several of these patients were also seen by a local doctor who also documented their progress.

The doctor has reported that all except one patient has had a marked clinical improvement following this treatment. Much of this improvement relates to a feeling of well being, increased energy levels and improved subjective sensory reports. A neurologist has confirmed that at least two of these patients have had significant objective responses which are unexpected in this group of patients and which cannot be put down as a placebo.

In addition, this group of patients have confirmed the total lack of any serum sickness or other significant side effect bar the red, inflamed and sometimes itchy response at the site of the subcutaneous injection which is never more than 1ml in volume.

## Claims

1. Use of a serum composition obtained from a goat after challenge with HIV in the preparation of a medicament for the treatment of a human for motor neurone disease, wherein the composition is administered by subcutaneous injection.

2. Use of a serum composition obtained from a goat after challenge with HIV in the preparation of a medicament for the treatment of a human for a chronic inflammatory condition of the nervous system.

3. The use of any of claims 1 to 2, wherein the composition comprises anti-HLA antibody.

4. The use of any of claims 1 to 3, wherein the composition is obtained from the serum of a goat after challenge with HIV lysate.

5. The use of any of claims 1 to 4, wherein the composition comprises an active compound derived from the blood of a suitably challenged goat by a serum extraction technique that is not designated to isolate individual, specific antibodies.

6. The use of any of claims 1 to 5, wherein the composition is administered weekly.

7. The use of any of claims 1 to 5, wherein the composition is administered monthly.

8. The use of claim 2, wherein the composition is administered by injection.

## Patentansprüche

1. Verwendung einer Serumzusammensetzung, die von einer Ziege nach der Provokation mit HIV erhalten worden ist, bei der Herstellung eines Medikaments für die Behandlung eines Menschen gegen Motoneuron-Krankheit, wobei die Zusammensetzung durch subkutanes Einspritzen verabreicht wird.

2. Verwendung einer Serumzusammensetzung, die von einer Ziege nach der Provokation mit HIV erhalten worden ist, bei der Herstellung eines Medikaments für die Behandlung eines Menschen gegen einen chronischen Entzündungszustand des Nervensystems.

3. Verwendung nach einem der Ansprüche 1 bis 2, wobei die Zusammensetzung Anti-HLA-Antikörper umfasst.

4. Verwendung nach einem der Ansprüche 1 bis 3, wobei die Zusammensetzung aus dem Serum einer Ziege nach der Provokation mit HIV-Lysat erhalten wird.

5. Verwendung nach einem der Ansprüche 1 bis 4, wobei die Zusammensetzung eine aktive Verbindung umfaßt, die aus dem Blut einer geeignet provozierten Ziege durch eine Serumextraktionstechnik derivatisiert wird, die nicht zum Isolieren einzelner spezifischer Antikörper bestimmt ist.

6. Verwendung nach einem der Ansprüche 1 bis 5, wobei die Zusammensetzung wöchentlich verabreicht wird.

7. Verwendung nach einem der Ansprüche 1 bis 5, wobei die Zusammensetzung monatlich verabreicht wird.

8. Verwendung nach Anspruch 2, wobei die Zusammensetzung durch Einspritzen verabreicht wird.

## Revendications

1. Utilisation d'une composition de sérum obtenue auprès d'une chèvre après provocation avec le VIH, dans la préparation d'un médicament pour le traitement d'un être humain contre une maladie du motoneurone, dans laquelle la composition est administrée par injection sous-cutanée.

2. Utilisation d'une composition de sérum obtenue auprès d'une chèvre après provocation avec le VIH, dans la préparation d'un médicament pour le traitement d'un être humain contre une affection inflammatoire chronique du système nerveux.

3. Utilisation selon l'une quelconque des revendications 1 à 2, dans laquelle la composition comprend un anticorps anti-HLA.

4. Utilisation selon l'une quelconque des revendications 1 à 3, dans laquelle la composition est obtenue à partir du sérum d'une chèvre après provocation avec un lysat de VIH.

5. Utilisation selon l'une quelconque des revendications 1 à 4, dans laquelle la composition comprend un composé actif dérivé du sang d'une chèvre provoquée comme il convient, par une technique d'extraction de sérum qui n'est pas élaborée pour isoler des anticorps spécifiques individuels.

6. Utilisation selon l'une quelconque des revendications 1 à 5, dans laquelle la composition est administrée hebdomadairement.

7. Utilisation selon l'une quelconque des revendications 1 à 5, dans laquelle la composition est administrée mensuellement.

8. Utilisation selon la revendication 2, dans laquelle la composition est administrée par injection.
